# EUROPEAN PATENT APPLICATION

(11) **EP 4 502 109 A1**
(43) Date of publication of application: **05.02.2025**
(21) Application number: 23775081.5
(22) Date of filing: 24.03.2023
(51) Int. Cl.: C09K 23/00, A61K 8/06, A61K 8/73, A61Q 19/00

(54) **EMULSIFIED COMPOSITION**

(30) Priority: 25.03.2022 JP 2022049699
(71) Applicant: House Wellness Foods Corporation, Hyogo 664-0011 (JP); House Foods Corporation, Higashi-Osaka-shi, Osaka 577-8520 (JP)
(72) Inventor: TOMOTAKE, Muneaki, Itami-shi, Hyogo 664-0011 (JP); KISHI, Takahiro, Itami-shi, Hyogo 664-0011 (JP); SASAKO, Hiroshi, Osaka (JP)
(74) Representative: Hoffmann Eitle
(86) International application number: PCT/JP2023/011763
(87) International publication number: WO 2023/182488

(57) **Abstract**

An object of the present invention is to provide an emulsified composition containing fat and oil, water, and cyclodextrin, and a water-soluble gelling agent, which has high emulsion stability. Provided is an emulsified composition containing water, fat and oil, cyclodextrin, and a prescribed water-soluble gelling agent, wherein a ratio of a blending amount of the fat and oil to a total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio.

## Description

### Technical Field

The present invention relates to an emulsified composition containing water, fat and oil, cyclodextrin, and a prescribed water-soluble gelling agent.

### Background Art

Emulsified compositions containing fat and oil, water, cyclodextrin, and a water-soluble gelling agent are widely used in various fields of, for example, pharmaceuticals, cosmetics, and foods and drinks.

Patent Literature 1 discloses an emulsified composition containing water, an oily component, α-cyclodextrin, and one or more of agar, low-strength agar, a combination of galactomannan or glucomannan and xanthan gum, carrageenan, furcelleran, gellan gum, and native gellan gum as gel-forming components.

Patent Literature 2 discloses an emulsified composition characterized by containing fat and oil, water, cyclodextrin, and the like, and also discloses that gums such as xanthan gum, guar gum, gum arabic CMC, carrageenan, and locust bean gum may be used as stabilizers.

Patent Literature 3 discloses an emulsified cosmetic obtained by mixing an aqueous raw material in which a water-soluble polymer compound such as pectin, tragacanth gum, gelatin, casein, sodium alginate, hydroxypropyl cellulose, hydroxyethyl cellulose, hydroxypropylmethyl cellulose, methyl cellulose, sodium polyacrylate, or carboxyvinyl polymer is dissolved with an oily raw material for a cosmetic, adding cyclodextrin thereto, and emulsifying the mixture.

Non Patent Literature 1 describes that emulsion stability increased when xanthan gum and tragacanth gum were each added as an emulsion stabilizer to an oil-drops-in-water type emulsion obtained by emulsifying a sample of soybean oil and an aqueous cyclodextrin solution at a volume ratio of 1:1.

### Citation List

### Patent Literature

Patent Literature 1: Japanese Patent Laid-Open No. 2016-204311
Patent Literature 2: Japanese Patent Laid-Open No. 10-262560
Patent Literature 3: Japanese Patent Publication No. 61-038166

### Non Patent Literature

Non Patent Literature 1: Nippon Shokuhin Kogyo Gakkaishi, Vol. 38, No. 1, 16-20 (1991)

### Summary of Invention

### Technical Problem

The present inventors have found that in a conventionally known emulsified composition containing fat and oil, water, cyclodextrin, and a water-soluble gelling agent, the oil and water may be easily separated from each other because emulsion stability of the emulsified composition is not sufficient.

Therefore, an object of the present invention is to provide an emulsified composition containing fat and oil, water, cyclodextrin, and a water-soluble gelling agent, which has high emulsion stability.

### Solution to Problem

As a result of intensive studies to solve the problem, the present inventors have found that an emulsified composition which has high emulsion stability can be obtained by blending at least one water-soluble gelling agent selected from the group consisting of carboxymethyl cellulose (CMC), xanthan gum, locust bean gum, guar gum, glucomannan, K-carrageenan, t-carrageenan, λ-carrageenan, tamarind gum, and gellan gum together with fat and oil, water, and cyclodextrin.

The present inventors have also found that when a mass ratio of a blending amount of the fat and oil to the total blending amount of the cyclodextrin and the water-soluble gelling agent (blending amount of the fat and oil:total blending amount of the cyclodextrin and the water-soluble gelling agent) is in a range of 1 or more and less than 9:9 or less and more than 1, the form (e.g., powder) obtained by drying the emulsified composition has excellent heat resistance and moisture resistance without discoloration or dissolution even in the case of being subjected to high temperature and high humidity conditions.

Furthermore, the present inventors have found that an emulsified composition obtained by re-emulsifying the dried form in a solvent such as water also maintains high emulsion stability.

The present invention is based on these new findings, and the following inventions are included.
[1] An emulsified composition, comprising water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum, wherein a ratio of a blending amount of the fat and oil to a total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio.
[2] The emulsified composition according to [1], wherein a content of the water is 10 mass% or less.
[3] The emulsified composition according to [1] or [2], which has a powder form.
[4] A re-emulsified composition, comprising the emulsified composition according to [2] or [3] and a solvent.
[5] The re-emulsified composition according to [4], further comprising an emulsifier.
[6] The re-emulsified composition according to [4] or [5], further comprising an aqueous organic solvent.
[7] A method for producing an emulsified composition, comprising mixing water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum to form an emulsified composition such that a ratio of a blending amount of the fat and oil to a total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio.
[8] The method for producing an emulsified composition according to [7], further comprising subjecting the obtained emulsified composition to a drying treatment.
[9] The method for producing an emulsified composition according to [8], wherein a content of the water in the emulsified composition is adjusted to 10 mass% or less by the drying treatment.
[10] The method for producing an emulsified composition according to [8] or [9], further comprising providing the obtained emulsified composition in a powder form.
[11] A method for producing a re-emulsified composition, comprising mixing the emulsified composition according to [2] or [3] with a solvent to form a re-emulsified composition.
[12] The method for producing a re-emulsified composition according to [11], further mixing an emulsifier.
[13] The method for producing a re-emulsified composition according to [11] or [12], further mixing an aqueous organic solvent.

The present specification encompasses the contents described in, for example, the specifications of Japanese Patent Application No. 2022-049699, filed on March 25, 2022, which is the basis of the priority of the present application.

All publications, patents, and patent applications cited herein are hereby incorporated by reference in their entirety.

### Advantageous Effects of Invention

According to the present invention, it is possible to provide an emulsified composition containing fat and oil, water, cyclodextrin, and a water-soluble gelling agent, which has excellent emulsion stability.

### Brief Description of Drawing

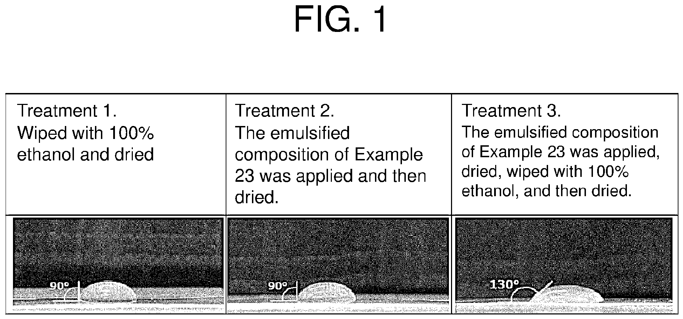

[Figure 1] Figure 1 is a photographic diagram showing a contact angle of liquid droplets (distilled water) dropped on the surface of polyethylene resin that has been subjected to various treatments 1 to 3: Treatment 1. The polyethylene resin is wiped with 100% ethanol and dried; Treatment 2. The emulsified composition of Example 23 is applied and then dried; and Treatment 3. The emulsified composition of Example 23 was applied, dried, wiped with 100% ethanol, and then dried.

### Description of Embodiments

The present invention relates to an emulsified composition containing water, fat and oil, cyclodextrin, and a prescribed water-soluble gelling agent.

As the "fat and oil" in the present invention, fat and oil commonly used in an emulsified composition is available, and both polar and non-polar fats and oils may be used. Examples of such fat and oil include, but are not limited to, plant-derived fat and oil (e.g., canola oil, refined rapeseed oil, soybean oil, corn oil, cottonseed oil, peanut oil, sesame oil, rice oil, rice bran oil, camellia oil, safflower oil, olive oil, linseed oil, Japanese basil oil, perilla oil, sunflower oil, palm oil, tea oil, coconut butter, avocado oil, *Aleurites moluccanus* seed oil, grapeseed oil, cocoa butter, coconut oil, wheatgerm oil, almond oil, evening primrose oil, castor oil, hazelnut oil, macadamia nut oil, rosehip oil, grape oil, cacao oil, jojoba oil, palm kernel oil, Japan wax, candelilla wax, and carnauba wax), paraffin, microcrystalline wax, ceresin, vaseline, ozokerite, synthetic ester oils such as isostearyl alcohol, caprylic alcohol, lauryl alcohol, stearyl alcohol, 2-octadecyl alcohol, myristyl alcohol, cetyl alcohol, phytosterol, cholesterol, stearic acid, isostearic acid, capric acid, lanolin acid, lauric acid, myristic acid, palmitic acid, behenic acid, linolic acid, linolenic acid, glyceryl monostearate, glyceryl monopalmitate, glyceryl monobehenate, glyceryl monomyristate, glyceryl monolaurate, glyceryl monolanolate, glyceryl monolinolate, glyceryl monolinolenate, glyceryl monooleate, glycerol triisostearate, isopropyl myristate, glycerol tri-2-heptylundecanoate, glycerol tri-2-ethylhexanoate, 2-heptylundecyl palmitate, di-2-heptylundecyl adipate, cetyl isooctanoate, trimethylolpropane-2-trimethylolheptylundecanoate, propane-2-ethylhexanoate, pentaerythritol-2-heptylundecanoate, pentaerythritol-2-ethylhexanoate, cholesterol isostearate, diethyl phthalate, and dibutyl phthalate, animal-derived fats and oils such as beef tallow, lard, lanolin, squalene, squalane, beeswax, and spermaceti, and silicone oil. In the present invention, the fat and oil is preferably animal-derived or plant-derived fat and oil with high safety and particularly preferably an edible vegetable oil that has been eaten and is highly safe. The fat and oil may be used alone or in combination with different fat and oil.

The emulsified composition of the present invention may contain fat and oil in any amount as long as the ratio of the blending amount of the fat and oil to the total blending amount of the cyclodextrin and the water-soluble gelling agent falls within a predetermined range in mass ratio, for example, in an amount of 1 mass% or more, 3 mass% or more, 5 mass% or more, or 7 mass% or more. The upper limit thereof is not particularly limited, but may be, for example, 50 mass% or less, 40 mass% or less, 30 mass% or less, 20 mass% or less, or 10 mass% or less. The range of the amount of fat and oil in the emulsified composition of the present invention can be expressed using two numerical values selected from each of the lower and upper numerical values. For example, the emulsified composition of the present invention may contain the fat and oil in an amount appropriately selected from the range of 1 mass% to 50 mass%, 1 mass% to 30 mass%, 1 mass% to 20 mass%, or 1 mass% to 10 mass%, preferably 5 mass% to 50 mass%, 5 mass% to 30 mass%, or 5 mass% to 20 mass%, and more preferably 5 mass% to 10 mass%, or 7 mass% to 10 mass%. If the amount of fat and oil is less than 1 mass%, emulsification may be insufficient, or the emulsified composition may give a creaky feeling when applied. On the other hand, if the amount of the fat and oil is more than 50 mass%, the emulsified composition may give strong oiliness such as stickiness or sliminess when applied. The composition may not provide a desired feeling of use in the utilization form in the intended application.

In the present specification, the amount of each component is expressed as an amount of mass%, where the amount of the emulsified composition of the present invention is 100 mass%.

In the emulsified composition of the present invention, water may be contained in any amount capable of emulsifying fat and oil to form an oil-in-water emulsion together with cyclodextrin and a prescribed water-soluble gelling agent. For example, the emulsified composition of the present invention may contain water in an amount of 15 mass% or more, 20 mass% or more, 30 mass% or more, 40 mass% or more, 45 mass% or more, 50 mass% or more, 60 mass% or more, or 70 mass% or more. The upper limit thereof is not particularly limited, but may be, for example, 90 mass% or less, or 85 mass% or less. The range of the amount of the water in the emulsified composition of the present invention can be expressed using two numerical values selected from each of the lower and upper numerical values. For example, the emulsified composition of the present invention may contain water in an amount appropriately selected from the range of 15 mass% to 90 mass%, 45 mass% to 90 mass%, or 70 mass% to 85 mass%. The amount of water contained in the emulsified composition of the present invention is preferably more than that of fat and oil in volume ratio. For example, the content of fat and oil and water can be 1: greater than 1 (fat and oil: water) in volume ratio, such as 1:2 or more, 1:3 or more, 1:4 or more, 1:5 or more, 1:6 or more, 1:10 or more, 1:15 or more, or 1:20 or more. The upper limit of the amount of water is not particularly limited, but can be 1:90 or less, 1:80 or less, 1:70 or less, 1:60 or less, or 1:50 or less. Through adjustment of the amounts of water and fat and oil contained in the emulsified composition of the present invention within the above ranges, the emulsified composition may provide a feeling of use of suppressed oiliness such as stickiness and sliminess.

"Cyclodextrin" means a cyclic non-reducing maltooligosaccharide having glucose as a constituent unit, and examples thereof include α-cyclodextrin having six glucose units, β-cyclodextrin having seven glucose units, and γ-cyclodextrin having eight glucose units. In the present invention, α-, β-, and γ-cyclodextrins, cyclodextrin derivatives, and any combination thereof can be used. Examples of the cyclodextrin derivatives include, but are not limited to, ethyl cyclodextrin, methyl cyclodextrin, hydroxyethyl cyclodextrin, hydroxypropyl cyclodextrin, methylamino cyclodextrin, amino cyclodextrin, carboxyethyl cyclodextrin, carboxymethyl cyclodextrin, sulfoxyethyl cyclodextrin, sulfoxyl cyclodextrin, acetyl cyclodextrin, branched cyclodextrin, cyclodextrin fatty acid ester, glucosyl cyclodextrin, and maltosyl cyclodextrin. Preferably, α-cyclodextrin is used. Since α-cyclodextrin has a high solubility in water, an emulsified composition with less roughness can be obtained.

The emulsified composition of the present invention may contain cyclodextrin in an amount capable of imparting emulsion stability to an oil-in-water emulsion together with a prescribed water-soluble gelling agent and providing a desired feeling of use in the utilization form in the intended application as long as the ratio of the blending amount of the fat and oil to the total blending amount of the cyclodextrin and the water-soluble gelling agent falls within a predetermined range in mass ratio. For example, the emulsified composition of the present invention may contain cyclodextrin in an amount of 0.5 mass% or more, 1 mass% or more, 2.5 mass% or more, 3 mass% or more, 4 mass% or more, 4.5 mass% or more, or 5 mass% or more. The upper limit thereof is not particularly limited, but may be, for example, 15 mass% or less, 10 mass% or less, 9 mass% or less, 8 mass% or less, 7 mass% or less, or 6 mass% or less. The range of the amount of the cyclodextrin in the emulsified composition of the present invention can be expressed using two numerical values selected from each of the lower and upper numerical values. For example, the emulsified composition of the present invention may contain the cyclodextrin in an amount appropriately selected from the range of 0.5 mass% to 15 mass%, for example, 1 mass% to 15 mass%, 2.5 mass% to 10 mass%, 2.5 mass% to 9 mass%, 3 mass% to 9 mass%, 4 mass% to 8 mass%, 4.5 mass% to 9 mass%, or 5 mass% to 7 mass%, and preferably 2.5 mass% to 9 mass% or 4.5 mass% to 9 mass%. If the amount of the cyclodextrin is less than 0.5 mass%, the emulsion stability of the composition may be insufficient. On the other hand, if the amount of the cyclodextrin is more than 15 mass%, the composition may have too high viscosity or give a strong creaky feeling. In either case, the composition may not provide a desired feeling of use in the utilization form in the intended application.

In the present invention, the "water-soluble gelling agent" generally means a substance that dissolves in water and imparts viscosity (sometimes also referred to as a thickener, a thickening stabilizer, or an adhesive, for example). Examples of the water-soluble gelling agent available in the present invention include carboxymethyl cellulose (CMC), xanthan gum, locust bean gum, guar gum, glucomannan, K-carrageenan, t-carrageenan, λ-carrageenan, tamarind gum, and gellan gum. Each of the water-soluble gelling agents may be used alone or in combination with different water-soluble gelling agents. More preferably, the water-soluble gelling agents are carboxymethyl cellulose (CMC), glucomannan, tamarind gum, and xanthan gum, which can impart particularly high emulsion stability to the emulsified composition of the present invention.

The emulsified composition of the present invention may contain a water-soluble gelling agent in an amount capable of imparting emulsion stability to an oil-in-water emulsion together with cyclodextrin and providing a desired feeling of use in the utilization form in the intended application as long as the ratio of the blending amount of the fat and oil to the total blending amount of the cyclodextrin and the water-soluble gelling agent falls within a predetermined range in mass ratio. For example, the emulsified composition of the present invention may contain the water-soluble gelling agent in an amount of 0.05 mass% or more, 0.1 mass% or more, 0.2 mass% or more, or 0.3 mass% or more. The upper limit thereof is not particularly limited, but may be, for example, 1 mass% or less, 0.8 mass% or less, 0.7 mass% or less, 0.6 mass% or less, or 0.5 mass% or less. The range of the amount of the water-soluble gelling agent in the emulsified composition of the present invention can be expressed using two numerical values selected from each of the lower and upper numerical values. For example, the emulsified composition of the present invention may contain the water-soluble gelling agent in an amount appropriately selected from the range of 0.05 mass% to 1 mass%, 0.1 mass% to 1 mass%, 0.2 mass% to 1 mass%, or 0.3 mass% to 1 mass%, preferably 0.2 mass% to 0.8 mass% or 0.2 mass% to 0.5 mass%, and more preferably 0.3 mass% to 0.8 mass% or 0.3 mass% to 0.5 mass%. If the amount of the water-soluble gelling agent is less than 0.1 mass%, the emulsion stability of the composition may be insufficient. On the other hand, if the amount of the water-soluble gelling agent is more than 1 mass%, the composition may have too high viscosity. In either case, the composition may not provide a desired feeling of use in the utilization form in the intended application.

In the emulsified composition of the present invention, the ratio of the blending amount of the fat and oil to the total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio. In the emulsified composition of the present invention, for example, the blending amount of the fat and oil: total blending amount of the cyclodextrin and the water-soluble gelling agent is 1:9, 2:8, 3:7, 4:6, 5:5, 6:4, 7:3, or 8:2, and preferably 5:5 in mass ratio. When the blending amount of the fat and oil is less than 1 and the total blending amount of the cyclodextrin and the water-soluble gelling agent is more than 9 in the ratio, emulsification and emulsion stability of the emulsified composition may be insufficient. In addition, when the blending amount of the fat and oil is 9 or more and the total blending amount of the cyclodextrin and the water-soluble gelling agent is 1 or less, the emulsified composition may give strong oiliness such as stickiness or sliminess, and in the form of a dried body, the emulsified composition does not have sufficiently high heat resistance or moisture resistance. In either case, the composition may not provide a desired feeling of use in the utilization form in the intended application.

In one embodiment, the emulsified composition of the present invention can contain the fat and oil in an amount appropriately selected from the range of 1 mass% to 50 mass%, preferably 1 mass% to 30 mass%, and more preferably 5 mass% to 30 mass%; the cyclodextrin in an amount appropriately selected from the range of 0.5 mass% to 15 mass%, preferably 2.5 mass% to 10 mass%, and more preferably 3 mass% to 10 mass%; and the water-soluble gelling agent in an amount appropriately selected from the range of 0.05 mass% to 1 mass%, preferably 0.1 mass% to 1 mass%, and more preferably 0.3 mass% to 1 mass%.

The emulsified composition of the present invention has high emulsion stability, and more preferably, the emulsified composition of the present invention has excellent emulsion stability with high heat resistance that allows the emulsified state to be maintained even after being subjected to high temperature (e.g., about 100°C to 130°C) conditions without causing demulsification.

The emulsified composition of the present invention has high emulsion stability regardless of the pH value, more preferably excellent emulsion stability with high heat resistance that allows the emulsified state to be maintained even after being subjected to high temperature (e.g., about 100°C to 130°C) conditions without causing demulsification.

The emulsified composition of the present invention is not limited to any particular form and may be provided in a form of a semi-solid/semi-liquid (e.g., a gel or a sol) or a dried body (e.g., a powder or a flake) prepared by reducing the water content, in addition to a form having the above-described water content. Such a form of a semi-solid/semi-liquid (e.g., a gel or a sol) or a dried body (e.g., a powder or a flake) can be formed after obtaining an emulsified composition in a form having the above water content by subjecting the emulsified composition to the conventionally known drying method to reduce the water content of the emulsified composition. Examples of such a drying method include, but are not limited to, freeze drying, heat drying, air drying, spray drying, drum drying, hot air drying, and vacuum drying. The water content of the emulsified composition after being subjected to the drying method can be appropriately selected depending on the intended form, and may be, for example, less than 15 mass%, 10 mass% or less, 5 mass% or less, or 1 mass% or less in the form of a dried body. The lower limit of the water content of the emulsified composition after being subjected to the drying method is not particularly limited but can be 0 mass% or more, more than 0 mass%, 0.1 mass% or more, 0.5 mass% or more, 0.6 mass% or more, 0.7 mass% or more, or 0.8 mass% or more. The range of the water content of the form of the dried body can be expressed using two numerical values selected from each of the lower and upper numerical values. For example, the water content of the emulsion composition may be 0 mass% to less than 15 mass%, more than 0 mass% and less than 15 mass%, 0.1 mass% to less than 15 mass%, 0.1 mass% to 10 mass%, 0.5 mass% to 5 mass%, 0.5 mass% to 1 mass%, 0.6 mass% to 1 mass%, 0.7 mass% to 1 mass%, or 0.8 mass% to 1 mass%. The emulsified composition after being subjected to the drying method may be used as it is or may be re-emulsified by adding an appropriate solvent (e.g., water, hot water, or a mixture of a water-soluble organic solvent and water) thereto/adding the composition to an appropriate solvent at the time of use (as used herein, sometimes referred to as a "re-emulsified composition").

The emulsified composition after being subjected to the drying method, particularly in the form of a dried body, has high heat resistance and high moisture resistance without discoloration or dissolution even after being subjected to high temperature (e.g., about 100°C to 200°C) conditions and/or high humidity (e.g., about 95% humidity) conditions.

The re-emulsified composition has high emulsion stability, more preferably excellent emulsion stability with high heat resistance that allows the emulsified state to be maintained even after being subjected to high temperature (e.g., about 100°C to 130°C) conditions without causing demulsification.

The re-emulsified composition has high emulsion stability regardless of the pH value, more preferably excellent emulsion stability with high heat resistance that allows the emulsified state to be maintained even after being subjected to high temperature (e.g., about 100°C to 130°C) conditions without causing demulsification.

In the present specification, unless otherwise specified, the "emulsified composition of the present invention" includes, in addition to the form having the above water content, the form of a semi-solid/semi-liquid (e.g., gel or sol) or a dried body (e.g., a powder or a flake), and the form of a re-emulsified composition.

The emulsified composition of the present invention can be used and contained as a base in applications for foods and beverages, cosmetics, topical drugs, pharmaceuticals (including quasi-drugs), chemical agents, chemical products, agrochemicals, toiletries, spray products, paints, industrial thin film materials, surface modifying materials, or the like, and can be provided in a utilization form (e.g., a form of a prescribed product or the like) in the intended application.

The emulsified composition of the present invention can exhibit excellent heat resistance, which allows the utilization form in the intended application using the emulsified composition of the present invention as a base to be subjected to high-temperature conditions in processes of production, sterilization, storage, use, or the like. For example, the utilization form of the emulsified composition of the present invention as a base in the intended application can be subjected to a heat sterilization treatment, more specifically to a high-temperature sterilization treatment (e.g., retort sterilization treatment) at 100°C to 130°C, thus providing a highly safe product with low/no antiseptic content. For example, the emulsified composition of the present invention can be subjected to high-temperature conditions together with other raw material or can be immediately combined with other raw materials subjected to high-temperature conditions, which not only contributes to the efficiency of the production process and operation but also enables the production of utilization forms (e.g., prescribed products) that could not be achieved before due to the avoidance of high-temperature conditions.

The emulsified composition of the present invention has high emulsion stability regardless of the pH value, more preferably excellent emulsion stability with high heat resistance that allows the emulsified state to be maintained even after being subjected to high temperature (e.g., about 100°C to 130°C) conditions without causing demulsification. The pH value of the emulsified composition of the present invention is not particularly limited and may be appropriately set to a desired pH value in the utilization form in the intended application. The pH value of the emulsified composition of the present invention can be adjusted using a conventionally known and generally used pH adjuster. In the present specification, the pH value of the emulsified composition of the present invention means a value measured at 25°C according to JIS Z8802: 2011. Accordingly, the utilization form in the intended application using the emulsified composition of the present invention as a base can be subjected to various pH conditions in processes of production, sterilization, storage, use, or the like, which enables the production of utilization forms (e.g., prescribed products) in various applications without being limited to a pH value.

In addition to the above-described components, the emulsified composition of the present invention may further contain, if necessary, components usually used in the production of the utilization form in the intended application, which can be appropriately blended in an amount according to the desired utilization form, as long as the effects of the present invention are not impaired. Examples of such components include, but are not limited to, excipients, disintegrators, lubricants, binders, diluents, buffers, suspending agents, thickeners, preservatives, antibacterial agents, antiseptics, antioxidants, ultraviolet absorbers, coloring agents, pigments, dyes, colorants, lubricants, plasticizers, solvents, solubilizers, isotonic agents, correctives, perfumes, sweeteners, taste components, acidulants, seasonings, humectants, vitamins, surfactants, chelating agents, antibacterial agents, emulsifiers, and water-soluble organic solvents.

In the emulsified composition of the present invention, the cyclodextrin may be used in combination with a water-soluble gelling agent to impart emulsion stability of an oil-in-water emulsion. The emulsion stability of the oil-in-water emulsion provided by the combined use of the cyclodextrin and the water-soluble gelling agent does not depend on a three-dimensional matrix gel formed by dissolving a general water-soluble gelling agent in water, followed by cooling (preferably, not including a three-dimensional matrix gel), but is considered to be due to the existence of an interaction by hydrogen bonds generated between the cyclodextrin and the water-soluble gelling agent, as will be described in detail by the following Examples. Since the emulsified composition of the present invention can impart high emulsion stability to the oil-in-water emulsion by the combined use of the cyclodextrin and the water-soluble gelling agent, the emulsified composition may be substantially free of an emulsifier, which is generally used in the production of a conventional emulsified composition, in addition to the cyclodextrin and the water-soluble gelling agent. In the present invention, the phrase "substantially free of an emulsifier" means that the emulsified composition of the present invention does not contain an emulsifier in such a manner as to exhibit an emulsifying effect, which is not intended to mean that the emulsified composition does not contain an emulsifier at all. The present invention can provide an advantageous emulsified composition with high safety and less irritation to the skin by being substantially free of an emulsifier, for example, when the emulsified composition of the present invention is applied to the skin. In addition, the emulsified composition of the present invention can provide a silky and moist feeling, for example, when the emulsified composition of the present invention is applied to the skin, by washing after application because the emulsified composition is substantially free of an emulsifier. Examples of the "emulsifier generally used in the production of a conventional emulsified composition" include, but are not limited to, glycerin fatty acid esters, organic acid monoglycerides, polyglycerin fatty acid esters, polyoxyethylene glycerin fatty acid esters, sorbitan fatty acid esters, propylene glycol fatty acid esters, polyoxyethylene fatty acid esters, polyglycerin condensed ricinoleic acid esters, sorbitan fatty acid esters, polyoxyethylene sorbitol fatty acid esters, sucrose fatty acid esters, lecithin, enzymatically decomposed lecithin, polyethylene glycol, and polypropylene glycol.

Alternatively, an emulsifier generally used in the production of a conventional emulsified composition may be appropriately selected and blended in the emulsified composition of the present invention according to the utilization form in the intended application, as long as the above-described desired effects of the present invention are not impaired. The emulsified composition of the present invention may contain the emulsifier in an amount capable of providing a desired feeling of use in the utilization form in the intended application as long as the above-described desired effects of the present invention are not impaired. For example, the emulsified composition of the present invention may contain an emulsifier in an amount of 0.005 mass% or more or 0.01 mass% or more. The upper limit thereof is not particularly limited, but may be, for example 0.5 mass% or less or 0.1 mass% or less. The range of the amount of the emulsifier in the emulsified composition of the present invention can be expressed using two numerical values selected from each of the lower and upper numerical values. For example, the emulsified composition of the present invention may contain the emulsifier in an amount appropriately selected from the range of 0.005 mass% to 0.5 mass% or less or 0.01 mass% to 0.1 mass%.

Adhesion of the emulsified composition can be suppressed/eliminated by blending the emulsifier in the emulsified composition of the invention.

The emulsified composition of the present invention is resistant to water-soluble organic solvents, thereby maintaining an emulsified state and excellent emulsion stability without causing demulsification in the presence of a water-soluble organic solvent, more preferably in the presence of a water-soluble organic solvent even after being subjected to high-temperature conditions.

As used herein, the term "water-soluble organic solvent" generally means an organic solvent which when mixed with water, dissolves in water to form a homogeneous phase without forming a two-phase system (consisting of an organic phase and an aqueous phase), and examples thereof include, but are not limited to, alcohols (e.g., methanol, ethanol, propanol, isopropanol, butanol, isobutanol, 2-butanol, or tert-butanol), polyalcohols (e.g., ethylene glycol, diethylene glycol, polyethylene glycol, propylene glycol, dipropylene glycol, polypropylene glycol, glycerin, or glucose), glycol ethers (e.g., ethylene glycol monomethyl ether, ethylene glycol monoethyl ether, diethylene glycol monomethyl ether, or diethylene glycol monoethyl ether), alkanediols (e.g., 1,2-butanediol, 1,3-propanediol, 1,3-butanediol, or 1,4-butanediol), amines (e.g., ethanolamine, diethanolamine, or triethanolamine), amides (e.g., formamide), ketones (e.g., acetone or acetylacetone), nitriles (e.g., acetonitrile), nitrogen-containing solvents (e.g., 2-pyrrolidone or triethanolamine), and sulfur-containing solvents (e.g., thiodiethanol or dimethyl sulfoxide). The water-soluble organic solvent can be appropriately selected according to the utilization form in the intended application of the emulsified composition of the present invention, and may be blended, for example, as a solvent for other components.

The emulsified composition of the present invention can be produced by mixing and stirring the water, fat and oil, cyclodextrin, and the prescribed water-soluble gelling agent, as well as other components, if necessary, in the above-described amounts. All of the components may be mixed and stirred together, or each component may be added separately or sequentially in any combination (in any order), then mixed and stirred. The emulsified composition obtained by mixing and stirring may be subjected to a heat sterilization treatment.

The emulsified composition obtained by mixing and stirring may be further subjected to a drying method, if necessary. The drying method can be performed by the conventionally known drying method, and the water content of the emulsified composition can be appropriately adjusted according to the intended form of a semisolid/semi-liquid (e.g., gel or sol) or a dried body. The resulting dried body may be further subjected to crushing, grinding, or abrasion treatment, if necessary, to be in the form of a powder, a flake, or the like. The emulsified composition obtained by being subjected to the drying method may be mixed and stirred with other components in the above-described amounts, if necessary, and/or subjected to a heat sterilization treatment.

The emulsified composition obtained by being subjected to the drying method can be used in the form of an oil-in-water emulsion without causing phase transition.

The emulsified composition obtained by being subjected to the drying method can be further mixed, stirred, and re-emulsified with an appropriate solvent, if necessary, to produce a re-emulsified composition. The solvent can be appropriately selected according to the utilization form in the intended application of the re-emulsified composition, and examples thereof include water and a mixture of a water-soluble organic solvent and water, which may be included in an amount appropriately selected from the range of 15 mass% to 90 mass%, preferably 45 mass% to 90 mass%, and more preferably 70 mass% to 85 mass%. The resulting re-emulsified composition may be mixed and stirred with other components in the above-described amounts, if necessary, and/or subjected to a heat sterilization treatment.

Hereinafter, the present invention will be described with reference to Examples, but the present invention is not limited to these Examples.

### Examples

### Experiment 1: Evaluation of Emulsion Stability of Emulsified Composition

### (1) Preparation of emulsified composition

According to the composition in Table 1 below, each component was added and mixed to prepare an emulsified composition. As the water-soluble gelling agent, any one of carboxymethyl cellulose (CMC), sodium alginate, gum arabic, glucomannan, guar gum, pectin, κ-carrageenan, tamarind gum, gellan gum, xanthan gum, t-carrageenan, locust bean gum, λ-carrageenan, distarch phosphate, hydroxypropyl starch, hydroxypropylated distarch phosphate, tragacanth gum, carboxyvinyl polymer, sodium polyacrylate, hydroxypropyl cellulose, methyl cellulose, hydroxypropylmethyl cellulose, and hydroxyethyl cellulose was blended.

Each component was mixed and stirred using a hand blender at 20,000 rpm for 10 minutes. Each composition (50 mL) obtained was transferred to a conical tube (Falcon (R) Conical Tube 50 mL) and centrifuged at 3,000 rpm for 1 minute. Subsequently, the thicknesses (depths) of water phase, micelles, and oil phase were visually observed, and the proportion of each phase (%) was measured, thereby evaluating the emulsion stability of each composition. The evaluation was made as "⊚" when the proportion of micelles was 100%, as "○" when the proportion of micelles was 70% or more and less than 100%, and as "×" when the proportion of micelles was less than 70%.

The amount of each component in the following table is expressed as an amount of mass%, where the amount of the obtained emulsified composition is 100 mass%. All of the fat and oil, α-cyclodextrin, and water-soluble gelling agents used were food additives (food grade).

**[Table 1]**

| Component | Blending amount (mass%) |
|---|---|
| Fat and oil (Canola oil + 1% oil red) | 25 |
| α-cyclodextrin | 2.5 |
| Water-soluble gelling agent | 0.35 |
| Water | 72.15 |
| Total | 100 |

### (2) Result

The water-soluble gelling agent added to the emulsified composition, the measured proportion of each phase, and the evaluation results are shown in Table 2. As is clear from the results, the emulsion stability of the emulsified composition varied depending on the water-soluble gelling agent added. When CMC, glucomannan, guar gum, κ-carrageenan, tamarind gum, gellan gum, xanthan gum, i-carrageenan, locust bean gum, or λ-carrageenan was added, the micelle phase was maintained and the emulsified composition exhibited high emulsion stability. In particular, when CMC, glucomannan, tamarind gum, or xanthan gum was added, separation of the water phase and the oil phase was not observed while remarkably high emulsion stability was confirmed.

**[Table 2]**

| Water-soluble gelling agent added | Proportion of each phase (%) | | | Emulsion stability |
|---|---|---|---|---|
| | Water phase | Micelle | Oil phase | |
| Carboxymethyl cellulose (CMC) | 0.00% | 100.00% | 0.00% | ⊚ |
| None | 0.00% | 70.27% | 29.73% | - |
| Sodium alginate | 32.90% | 67.10% | 0.00% | × |
| Gum arabic | 50.00% | 15.63% | 34.38% | × |
| Glucomannan | 0.00% | 100.00% | 0.00% | ⊚ |
| Guar gum | 0.00% | 91.11% | 8.89% | O |
| Pectin | 66.67% | 26.67% | 6.67% | × |
| κ-carrageenan | 6.67% | 93.33% | 0.00% | O |
| Tamarind gum | 0.00% | 100.00% | 0.00% | ⊚ |
| Gellan gum | 8.11% | 91.89% | 0.00% | O |
| Xanthan gum | 0.00% | 100.00% | 0.00% | ⊚ |
| 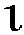 -carrageenan | 2.27% | 97.73% | 0.00% | O |
| Locust bean gum | 0.00% | 97.06% | 2.94% | O |
| λ-carrageenan | 4.44% | 95.56% | 0.00% | O |
| Distarch phosphate | 69.70% | 24.24% | 6.06% | × |
| Hydroxypropyl starch | 69.70% | 24.24% | 6.06% | × |
| Hydroxypropylated distarch phosphate | 69.70% | 24.24% | 6.06% | × |
| Tragacanth gum | 20.00% | 40.00% | 40.00% | × |
| Carboxylvinyl polymer | 28.50% | 28.50% | 43.00% | × |
| Sodium polyacrylate | 0.00% | 60.00% | 40.00% | × |
| Hydroxypropyl cellulose | 60.00% | 5.00% | 35.00% | × |
| Methyl cellulose | 60.00% | 0.00% | 40.00% | × |
| Hydroxypropylmethyl cellulose | 60.00% | 0.00% | 40.00% | × |
| Hydroxyethyl cellulose | 60.00% | 0.00% | 40.00% | × |

### Experiment 2: Evaluation of Interaction between α-Cyclodextrin and Water-Soluble Gelling Agent in Emulsified Composition

To evaluate the interaction between α-cyclodextrin and a water-soluble gelling agent in an aqueous solution, enthalpy change (ΔH) was determined by isothermal titration calorimetry (ITC) in accordance with a conventionally known method. Specifically, using an isothermal titration calorimeter (NANO ITC SV; TA Instruments), 10 µL of an aqueous solution of water-soluble gelling agent (0.05 g/100 mL) was added dropwise to an aqueous solution of α-cyclodextrin(25 g/100 mL) 25 times at intervals of 180 seconds (75 minutes) to determine an enthalpy value (µJ) from the area of the titration peak at the 25th (final) titration.

The results are shown in Table 3. Positive enthalpy values, i.e., endothermic reactions, were observed only when CMC and xanthan gum, which had high emulsion stability in Experiment 1, were used. This result suggests the presence of an interaction between the water-soluble gelling agent and α-cyclodextrin via hydration water, and it is considered that, for example, a part of water molecules hydrated in the water-soluble gelling agent forms a hydrogen bond with α-cyclodextrin. A hydrogen bond formed between water molecules usually has a shorter distance and a higher energy value than those of a hydrogen bond formed between the water-soluble gelling agent and α-cyclodextrin, which causes intermolecular repulsion due to a charged site. Therefore, when a part of water molecules hydrated in the water-soluble gelling agent forms a hydrogen bond with α-cyclodextrin, it is considered that an endothermic reaction occurs as a result of the subtraction of energy values. The heat transfer (positive enthalpy value) suggests the presence of the interaction between the water-soluble gelling agent and α-cyclodextrin, and this interaction is considered to be a factor in giving high emulsion stability in Experiment 1.

**[Table 3]**

| Water-soluble gelling agent | Enthalpy value (µJ) |
|---|---|
| Carboxymethyl cellulose (CMC) | 247.1 |
| Xanthan gum | 326.1 |
| Sodium alginate | -12.15 |
| Gum arabic | -25.36 |
| Pectin | -13.45 |
| Distarch phosphate | -14.78 |
| Hydroxypropyl starch | -18.96 |
| Hydroxypropylated distarch phosphate | -15.31 |
| Tragacanth gum | -20.08 |
| Carboxylvinyl polymer | -23.53 |
| Sodium polyacrylate | -22.36 |
| Hydroxypropyl cellulose | -11.46 |
| Methyl cellulose | -18.04 |
| Hydroxypropylmethyl cellulose | -16.69 |
| Hydroxyethyl cellulose | -13.76 |
| Water-water | -19.23 |

### Experiment 3: Performance Evaluation of Emulsified Composition (Texture)

### (1) Preparation of emulsified composition

According to the composition in Table 4 below, each component was added and mixed to prepare emulsified compositions of Examples 1 to 4 and Comparative Examples 1 to **4.** Each component was mixed and stirred using a hand blender at 20,000 rpm for 10 minutes. Each emulsified composition obtained was evaluated for the following evaluation items based on the criteria. The evaluation was made on four human subjects.

Viscosity (creaminess): when the emulsified compositions are each spread on the palm, "○" in the case of having a good feeling of use such that the cream is lightly spread; "△" in the case of having a feeling of use such that the cream is spread; and "X" in the case of providing no feeling of use such that the cream is spread (too low viscosity or too high viscosity).

Emulsifying ability after storage: "O" in the case where emulsification is maintained after storage at 40°C for 7 days; "X" in the case where separation of water and oil is observed.

Slimy feeling immediately after application: immediately after application to the palm, "○" in the case of the absence of oiliness (stickiness or sliminess); and "×" in the case of the presence of oiliness.

Feeling of use after washing: when the emulsified compositions are each applied to the palm and then washed, "○" in the case where a silky and moist feeling is recognized, "△" in the case where a silky and moist feeling is recognized with slight oiliness at the same time, and "X" in the case where a silky and moist feeling is not recognized. Although the same result can be obtained when only water is used or when hand soap is used for washing, the hand soap was used for washing in this experiment.

Heat sterilization: "O" in the case where emulsification is maintained after retort sterilization (123°C, 50 minutes), followed by cooling to room temperature, "X" in the case where separation of water and oil is observed.

The amount of each component in the following table is expressed as an amount of mass%, where the amount of the obtained emulsified composition is 100 mass%.

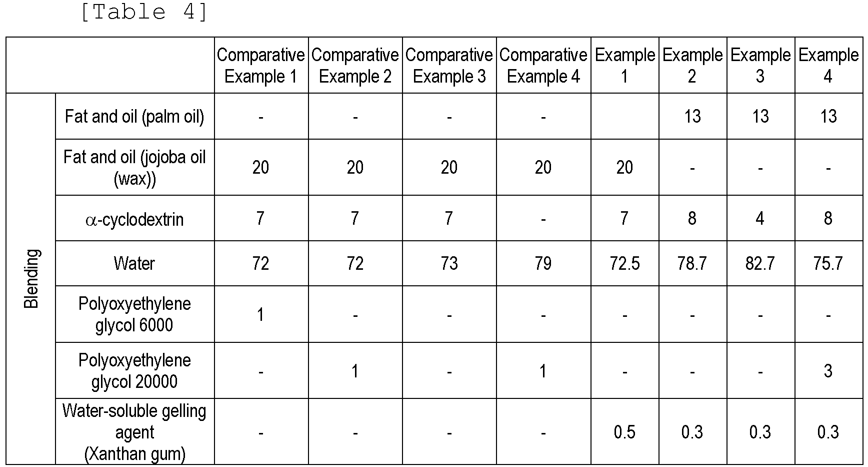

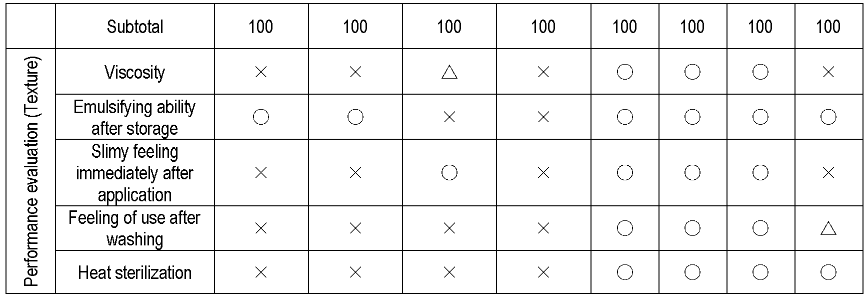

### (2) Result

The results of each evaluation for each emulsified composition obtained are shown in Table 4.

From the results of Examples 1 to 4, it was confirmed that the fat and oil is not limited to specific ones, and various fat and oil can be used.

When polyoxyethylene glycol was used instead of the water-soluble gelling agent (Comparative Examples 1, 2, and 4), some of the following were observed: a lack of a feeling of use such that the cream was spread, a decrease in emulsifying ability after storage, a decrease in the feeling of use immediately after application and after washing, and a decrease in emulsifying ability after heat sterilization, and it was confirmed that the performance was inferior to that of the emulsified compositions of Examples 1 to 4 using the water-soluble gelling agent.

Although the emulsified compositions of Examples had a good feeling of use such that the cream was lightly spread, the emulsified compositions of Examples 1 to 3 had particularly excellent performance and showed good results in all evaluation items. In Examples 1 to 3, Examples 1 and 2 in which the content of α-cyclodextrin was relatively high gave a slight feeling of tightness (creaky feeling) when applied as compared with Example 3 in which the content of α-cyclodextrin was relatively low, but not to the extent of interfering with the good feeling of use.

### Experiment 4: Powder Emulsified Composition (Freeze-Dried)

### (1) Preparation of powder emulsified composition

According to the composition in Table 5 below, each component was added and mixed to prepare an emulsified composition. Each component was mixed and stirred using a hand blender at 20,000 rpm for 5 minutes. Next, the resulting emulsified composition was freeze-dried (vacuum state after freezing, followed by freeze-drying at 20°C for 16 hours) and crushed to obtain a powder emulsified composition.

The amount of each component in the following table is expressed as an amount of mass%, where the amount of the obtained emulsified composition is 100 mass%.

**[Table 5]**

| Component | Blending amount (mass%) |
|---|---|
| Fat and oil (palm oil) | 13 |
| α-cyclodextrin | 8 |
| Water-soluble gelling agent (CMC) | 0.3 |
| Water | 78.7 |
| Total | 100 |

### (2) Performance evaluation of powder emulsified composition (texture)

(i) The water content of the obtained powder emulsified composition was measured by a normal pressure heating and drying method in accordance with an ordinary method. Specifically, the obtained emulsified composition was put in a weighing can and precisely weighed (mass before drying), dried at 105°C for 5 hours, then transferred to a desiccator, allowed to cool for 1 hour, and precisely weighed (mass after drying). The water content (mass%) of the powder emulsified composition was determined from the mass before drying and the mass after drying.
(ii) The obtained powder emulsified composition was evaluated for "viscosity (creaminess)", "emulsification ability after storage", "slimy feeling immediately after application", and "feeling of use after washing" by the same method and criteria as those described in "Experiment 3: Performance Evaluation (Texture)". As a control, a composition obtained by the same operation was used, except that each component was added according to the composition in Table 5 and mixed by hand without applying a shear force (i.e., an emulsified composition was not prepared) to prepare the composition.

### (3) Result

(i) The measurement results of water content are shown in Table 6. The water content was measured using two powder emulsified compositions individually prepared as samples. The water contents of the obtained powder emulsified compositions were about 0.7 mass% and about 0.9 mass%, and the average was about 0.8 mass%.

**[Table 6]**

| Sample | Weighing can mass (g) | Mass before drying (g) | Mass after drying (g) | Water content (mass%) (Average value) | |
|---|---|---|---|---|---|
| No. 1 | 16.4025 | 16.9279 | 16.924 | 0.742 | 0.807 |
| No. 2 | 17.0752 | 17.9472 | 17.9396 | 0.872 | |

| | | | | | |
|---|---|---|---|---|---|
| **The mass before drying and the mass after drying indicate the total mass of the weighing can and the powder emulsified composition.** | | | | | |

(ii) The powder emulsified composition had a form of a free-flowing powder, whereas the control composition had a form of a paste in which fat and oil as liquids and a powder were mixed together, and thus a form of a free-flowing powder was not obtained.

The obtained powder emulsified composition was evaluated as "○" in all of "viscosity (creaminess)", "emulsification ability after storage", "slimy feeling immediately after application", and "feeling of use after washing". Similarly to the emulsified composition of Experiment 3, the powder emulsified composition had a good feeling of use, in which the composition could be spread in a transparent state such that an oil film was quickly formed when spread on the palm. The powder emulsified composition also simultaneously provided a smooth feeling as if a powder was applied and a moist feeling as if a cream was applied when washed after application. On the other hand, the control composition did not provide a good feeling of use when applied to the palm because the liquid and the solid were separated from each other, giving a rough and creaky feeling.

### Experiment 5: Powder Emulsified Composition (Spray-Dried)

### (1) Preparation of powder emulsified composition

According to the composition in Table 5, each component was added and mixed to prepare an emulsified composition. Each component was mixed and stirred using a hand blender at 20,000 rpm for 5 minutes. Next, the resulting emulsified composition was spray-dried (190°C) to obtain a powder emulsified composition.

### (2) Performance evaluation of powder emulsified composition (texture)

(i) The water content (mass%) of the obtained powder emulsified composition was determined in the same manner as described in "Experiment 4: Powder Emulsified Composition (Freeze-dried)".
(ii) The obtained powder emulsified composition was evaluated for "viscosity (creaminess)", "emulsification ability after storage", "slimy feeling immediately after application", and "feeling of use after washing" in the same manner as described in "Experiment 4: Powder Emulsified Composition (Freeze-dried)".

### (3) Result

(i) The measurement results of water content are shown in Table 7. The water content was measured using two powder emulsified compositions individually prepared as samples. The water contents of the obtained powder emulsified compositions were about 0.7 mass% and about 1.0 mass%, and the average was about 0.8 mass%.

**[Table 7]**

| Sample | Weighing can mass (g) | Mass before drying (g) | Mass after drying (g) | Water content (mass%) (Average value) | |
|---|---|---|---|---|---|
| No. 1 | 16.125 | 18.2926 | 18.2717 | 0.964 | 0.809 |
| No. 2 | 16.9779 | 18.923 | 18.9103 | 0.653 | |

| | | | | | |
|---|---|---|---|---|---|
| The mass before drying and the mass after drying indicate the total mass of the weighing can and the powder emulsified composition. | | | | | |

(ii) Similarly to the freeze-dried composition, the spray-dried powder emulsified composition also had a form of free-flowing powder and was evaluated as "O" in all of "viscosity (creaminess)", "emulsification ability after storage", "slimy feeling immediately after application", and "feeling of use after washing". The spray-dried powder emulsified composition had a good feeling of use, in which the composition could be spread in a transparent state such that an oil film was quickly formed when spread on the palm. The powder emulsified composition also simultaneously provided a smooth feeling as if a powder was applied and a moist feeling as if a cream was applied when washed after application. It was confirmed that even when the drying treatment was performed in a different manner, the same characteristics were obtained.

The results revealed that an emulsified composition having high emulsion stability and a good cream-like texture can be obtained by blending cyclodextrin and a prescribed water-soluble gelling agent in combination with an oil-in-water emulsion containing water and fat and oil. It has been confirmed that when the emulsified composition is spread, the feeling of use is unprecedented, and the emulsified composition has a good cream-like texture and simultaneously provided a smooth feeling as if a powder was applied and a moist feeling as if a cream was applied when washed with water or the like after application.

Further, it was confirmed that a dried form (powder emulsified composition) obtained by subjecting the emulsified composition to a drying treatment also could be spread in a transparent state such that an oil film was quickly formed when spread, and thus provided the same characteristics. On the other hand, these characteristics were not obtained for the dried form obtained by subjecting the emulsified composition to a drying treatment without passing through the emulsified composition having the high emulsion stability. Therefore, the good characteristics in dried form are also due to the maintenance of the emulsified state without solid-liquid separation, which is attributed to the high emulsion stability of the emulsified composition before being subjected to a drying treatment.

### Experiment 6: Evaluation of Heat Resistance of Powder Emulsified Composition

### (1) Preparation of powder emulsified composition

According to the composition in Tables 8 to 10 below, each component was added and mixed to prepare an emulsified composition. Each component was mixed and stirred using a hand blender at 20,000 rpm for 5 minutes. Next, the resulting emulsified composition was freeze-dried (vacuum state after freezing, followed by freeze-drying at 20°C for 16 hours) and crushed to obtain white powder emulsified compositions.

### (2) Heat resistance test of powder emulsified composition

The obtained powder emulsified compositions were each put in a dry heat sterilizer (manufactured by HIRAYAMA Manufacturing Corporation) and heat-treated at 105°C (product temperature) for 13 hours. After completion of the heat treatment, each of the powder emulsified compositions was removed from the dry heat sterilizer and allowed to cool to room temperature, and the changes in the powder emulsified compositions were visually evaluated. As a control, conventionally known white powdered fats and oils (N Neopowder A and N Neopowder P (NOF CORPORATION)) were used and heat-treated in the same manner, and the change thereof was visually evaluated.

### (3) Result

The evaluation results of the heat resistance of the respective powder emulsified compositions are also shown in Tables 8 to 10 below.

Tables 8 to 10 also show the measurement results of the water content (mass%) of the powder emulsified composition after being subjected to the heat treatment, which was determined in the same manner as described in "Experiment 4: Powder Emulsified Composition (Freeze-dried)".

The amount of each component in the tables is expressed as an amount of mass%, where the amount of the obtained emulsified composition is 100 mass%. The "mass before drying" and the "mass after drying" indicate the total mass of the weighing can and the powder emulsified composition.

**[Table 8]**

| | | Example 21 | Example 22 | Example 23 | Example 24 | Comparative Example 25 |
|---|---|---|---|---|---|---|
| Blending | Fat and oil (palm oil) | 1 | 3 | 5 | 7 | 9 |
| | α-cyclodextrin | 8.7 | 6.7 | 4.7 | 2.7 | 0.7 |
| | Water-soluble gelling agent (CMC) | 0.3 | 0.3 | 0.3 | 0.3 | 0.3 |
| | Water | 90 | 90 | 90 | 90 | 90 |
| | Total | 100 | 100 | 100 | 100 | 100 |
| Fat and oil: α - CD + CMC (mass ratio) | | 1:9 | 3:7 | 5:5 | 7:3 | 9:1 |
| Measurement result of water content | Weighing can mass (g) | 16.9097 | 16.8938 | 16.7535 | 16.7441 | 16.51 |
| | Mass before drying (g) | 17.5318 | 17.382 | 17.4144 | 18.0878 | 18.0576 |
| | Mass after drying (g) | 17.5355 | 17.3835 | 17.4177 | 18.0946 | 18.0553 |
| | Water content (mass%) | -0.595 | -0.307 | -0.499 | -0.506 | 0.149 |
| Evaluation result of heat resistance | | White powder with no change | White powder with no change | White powder with no change | Slight yellowing observed, but no change | Yellowing, with strong properties of fat and oil rather than powder, resulting in stickiness |

**[Table 9]**

| | | Example 23 | Example 26 | Example 27 | Example 28 | Example 29 |
|---|---|---|---|---|---|---|
| Blending | Fat and oil (palm oil) | 5 | 5 | 5 | 5 | 5 |
| | α-cyclodextrin | 4.7 | 4.7 | 4.7 | 4.7 | 4.7 |
| | Water-soluble gelling agent (CMC) | 0.3 | 0.05 | 0.1 | 0.5 | 1 |
| | Water | 90 | 90.25 | 90.2 | 89.8 | 89.3 |
| | Total | 100 | 100 | 100 | 100 | 100 |
| | Weighing can mass (g) | 16.9097 | 16.876 | 16.8669 | 16.7719 | 16.4951 |

| | | | | | | |
|---|---|---|---|---|---|---|
| Measurement result of water content | Mass before drying (g) | 17.5318 | 18.3507 | 18.2883 | 17.5527 | 17.3018 |
| | Mass after drying (g) | 17.5355 | 18.3591 | 18.2957 | 17.5532 | 17.3013 |
| | Water content (mass%) | -0.595 | -0.570 | -0.521 | -0.064 | 0.062 |
| Evaluation result of heat resistance | | White powder with no change | White powder with no change | White powder with no change | White powder with no change | White powder with no change |

**[Table 10]**

| | | Example 23 | Example 210 | Example 211 | Comparative Example 212 | Comparative Example 213 |
|---|---|---|---|---|---|---|
| | Fat and oil (palm oil) | 5 | 5 | 5 | 5 | 5 |
| | α-cyclodextrin | 4.7 | 4.7 | 4.7 | 4.7 | 4.7 |
| | CMC | 0.3 | 0 | 0 | 0 | 0 |
| | Xanthan gum | 0 | 0.3 | 0 | 0 | 0 |
| Blending | Tamarind gum | 0 | 0 | 0.3 | 0 | 0 |
| | Gum arabic | 0 | 0 | 0 | 0.3 | 0 |
| | Dextrin | 0 | 0 | 0 | 0 | 0.3 |
| | Water | 90 | 90 | 90 | 90 | 90 |
| | Total | 100 | 100 | 100 | 100 | 100 |
| Measurement result of water content | Weighing can mass (g) | 16.9097 | 16.8492 | 17.0118 | 16.5018 | 16.7658 |
| | Mass before drying (g) | 17.5318 | 17.6849 | 17.7749 | 17.3055 | 17.8322 |
| | Mass after drying (g) | 17.5355 | 17.6854 | 17.7786 | 17.304 | 17.8327 |
| | Water content (mass%) | -0.595 | -0.060 | -0.485 | 0.187 | -0.047 |
| Evaluation result of heat resistance | | White powder with no change | White powder with no change | White powder with no change | Yellowing observed | Yellowing, with strong properties of fat and oil rather than powder, resulting in some adhesion |

The results confirmed that a dried form (powder emulsified composition) obtained by subjecting the emulsified composition obtained by blending cyclodextrin and a prescribed water-soluble gelling agent in combination in an oil-in-water emulsion containing water and fat and oil to a drying treatment has excellent heat resistance without dissolving or yellowing even when subjected to high temperature conditions. Such excellent heat resistance was also confirmed when the powder emulsified composition of Example 23 was further subjected to a heat treatment under a high temperature condition (200°C (product temperature) for 13 hours).

On the other hand, it was confirmed that all of the conventionally known powdered fats and oils used as a control discolored entirely to deep yellow upon heat treatment, failed to maintain the powder form due to sticking, and had poor heat resistance.

Comparative Example 25 is an example in which the ratio of the blending amount of the fat and oil to the total blending amount of the α-cyclodextrinand the water-soluble gelling agent (blending amount of the fat and oil: total blending amount of the α-cyclodextrinand the water-soluble gelling agent) is 9:1 in mass ratio, and it was confirmed that heat treatment under high temperature conditions resulted in an overall yellow color, strong appearance of the properties of fats and oils causing stickiness, adhesion causing lumps, the form of powder was not maintained, and heat resistance was poor (Table 8).

Comparative Examples 212 and 213 are examples of using "gum arabic" and "dextrin", which are not included in the prescribed water-soluble gelling agent in the present invention, and it was confirmed that the form of the powder before heat treatment was not maintained and heat resistance was poor, as yellowing and properties of fats and oils were strongly expressed, causing stickiness (Table 10).

### Experiment 7: Evaluation of Moisture Resistance of Powder Emulsified Composition

### (1) Moisture resistance test of powder emulsified composition

Each of the powder emulsified compositions (Examples 21 to 211, Comparative Examples 25 to 213) prepared in "Experiment 6: Evaluation of Heat Resistance of Powder Emulsified Composition" and the conventionally known white powdered fats and oils (N Neopowder A and N Neopowder P (NOF CORPORATION)) were left in a constant temperature and humidity test chamber (PR-4KP, manufactured by ESPEC CORP.) at 40°C and 90% humidity for 1 week for humidification. After completion of the treatment, each of the powder emulsified compositions was removed from the constant temperature and humidity test chamber and allowed to cool to room temperature, and the changes in the powder emulsified compositions were visually evaluated.

### (2) Result

The evaluation results of the moisture resistance of the respective powder emulsified compositions are shown in Tables 11 to 13 below.

Table 11 also shows the measurement results of the water content (mass%) of the powder emulsified composition after being subjected to the moisture treatment, which was determined in the same manner as described in "Experiment 4: Powder Emulsified Composition (Freeze-dried)".

In the table, the "mass before drying" and the "mass after drying" indicate the total mass of the weighing can and the powder emulsified composition.

**[Table 11]**

| | | Example 21 | Example 22 | Example 23 | Example 24 | Comparative Example 25 |
|---|---|---|---|---|---|---|
| Measurement result of water content | Weighing can mass (g) | 11.3357 | 10.4124 | 11.0854 | 10.9957 | 11.3154 |
| | Mass before drying (g) | 12.3476 | 11.4237 | 12.0899 | 11.9855 | 12.3234 |
| | Mass after drying (g) | 12.3551 | 11.4258 | 12.0954 | 11.9904 | 12.3284 |
| | Water content (mass%) | 0.741 | 0.208 | 0.548 | 0.495 | 0.496 |
| Evaluation result of moisture resistance | | White powder with no change | White powder with no change | White powder with no change | White powder with no change | Yellowing and caking |

**[Table 12]**

| | | Example 26 | Example 27 | Example 28 | Example 29 |
|---|---|---|---|---|---|
| Measurement result of water content | Weighing can mass (g) | 10.4728 | 10.8877 | 10.6533 | 11.4528 |
| | Mass before drying (g) | 11.5419 | 11.8786 | 11.6726 | 12.4348 |
| | Mass after drying (g) | 11.5482 | 11.8852 | 11.6784 | 12.4398 |
| | Water content (mass%) | 0.589 | 0.666 | 0.569 | 0.509 |
| Evaluation result of moisture resistance | | White powder with no change | White powder with no change | White powder with no change | White powder with no change |

**[Table 13]**

| | | Example 210 | Example 211 | Comparative Example 212 | Comparative Example 213 |
|---|---|---|---|---|---|
| Measurement result of water content | Weighing can mass (g) | 10.8547 | 10.8967 | 10.0238 | 11.8972 |
| | Mass before drying (g) | 11.8436 | 11.8684 | 11.0189 | 12.8157 |
| | Mass after drying (g) | 11.8486 | 11.8724 | 11.0297 | 12.8391 |
| | Water content (mass%) | 0.506 | 0.412 | 1.085 | 2.548 |
| Evaluation result of moisture resistance | | White powder with no change | White powder with no change | Some yellowing and caking | Yellowing and caking |

The results confirmed that a dried form (powder emulsified composition) obtained by subjecting the emulsified composition obtained by blending cyclodextrin and a prescribed water-soluble gelling agent in combination in an oil-in-water emulsion containing water and fat and oil to a drying treatment has excellent moisture resistance without yellowing or caking even when subjected to high humidity conditions.

On the other hand, it was confirmed that all of the conventionally known powdered fats and oils used as a control discolored entirely to deep yellow upon moisture treatment, were highly hygroscopic and thereby caked, resulting in failure to maintain the powder form, and had poor moisture resistance.

Comparative Example 25 is an example in which the ratio of the blending amount of the fat and oil to the total blending amount of the α-cyclodextrin and the water-soluble gelling agent (blending amount of the fat and oil: total blending amount of the α-cyclodextrin and the water-soluble gelling agent) is 9:1 in mass ratio, and it was confirmed that the moisture treatment resulted in an overall yellow color, caking to form lumps, failure to maintain the form of powder, and poor moisture resistance (Table 11).

Comparative Examples 212 and 213 are examples of using "gum arabic" and "dextrin", which are not included in the prescribed water-soluble gelling agent in the present invention, and it was confirmed that the form of the powder before moisture treatment was not maintained and moisture resistance was poor, as yellowing and high hygroscopicity, resulting in caking (Table 13).

### Experiment 8; Re-emulsified Composition of Powder Emulsified Composition

### (1) PH resistance test and heat resistance test

The powder emulsified composition of Example 23 prepared in "Experiment 6: Evaluation of Heat Resistance of Powder Emulsified Composition" above was added to water in a glass beaker to a concentration of 10 mass%, mixed, and re-emulsified to prepare an emulsified composition. The powder emulsified composition was mixed with water by stirring at 300 rpm for 1 hour at room temperature using a magnetic stirrer (RS-4AN, manufactured by AS ONE Corporation). Subsequently, the resulting re-emulsified compositions were each adjusted to have a pH value (25°C) of 2, 3, 4, 7, 9, and 10 using a potassium phosphate buffer, and the demulsification state (or emulsion stability) of each of the re-emulsified compositions and the occurrence of adhesion to the glass beaker wall surface were visually evaluated. The demulsification state (or emulsion stability) was evaluated according to the following criteria.

### <Evaluation criteria>

⊚: No change. Emulsification was maintained.
○: No demulsification occurred, but some oil floating or separated-out water was observed.
×: Separation of oil and water layers was observed. Demulsification occurred.

In addition, 30 mL of the re-emulsified composition adjusted to each of the above pH values (25°C) was put in a 100 mL beaker and heat-treated in a water bath at 90°C (product temperature) for 5 minutes. After the heat treatment, the emulsified compositions were removed from the water bath and allowed to stand at room temperature for 30 minutes. Then, the demulsification state (or emulsion stability) of each of the emulsified compositions was visually evaluated according to the above criteria.

### (2) Result

**[Table 14]**

| Re-emulsified composition of powder emulsified composition | | | | | | |
|---|---|---|---|---|---|---|
| pH value (25°C) | 2 | 3 | 4 | 7 | 9 | 10 |
| Evaluation of emulsion stability | ⊚ | ⊚ | ⊚ | ⊚ | ⊚ | ⊚ |
| Occurrence of adhesion to the wall surface | Occurred | Occurred | Occurred | Occurred | Occurred | Occurred |
| Evaluation of emulsion stability after heat treatment | ⊚ | ⊚ | ⊚ | ⊚ | ⊚ | ⊚ |

It was confirmed that a re-emulsified composition prepared by adding the powder emulsified composition to water, followed by re-emulsification, maintained an emulsified state at any pH value (25°C) of 2,3,4,7,9 and 10 and did not cause demulsification, indicating that the re-emulsified composition had excellent emulsion stability with high pH resistance.

Regardless of the pH value (25°C), much adhesion was observed on the wall surface of the housed glass beaker for all of the re-emulsified compositions. Such a re-emulsified composition may be useful in applications where adhesion is required, such as, but not limited to, paints.

Regardless of the pH value (25°C), it was confirmed that the emulsified state was maintained without causing demulsification after the heat treatment for all of the re-emulsified compositions, revealing that the re-emulsified compositions had high heat resistance and excellent emulsion stability.

### Experiment 9: Re-emulsified Composition with Added Emulsifier

### (1) Various resistance tests

Sucrose palmitate (P-1670, manufactured by Mitsubishi Chemical Corporation) was added as an emulsifier in an amount of 0.01 mass% to re-emulsified compositions having a pH value (25°C) of 2, 3, 4, 7, 9, or 10, each of which was prepared in "Experiment 8; Re-emulsified Composition of Powder Emulsified Composition" above, and then mixed and stirred at room temperature for 1 hour. Each of the obtained re-emulsified compositions was evaluated for the demulsified state (or emulsion stability), the occurrence of adhesion to the wall surface of the glass beaker, and the demulsified state (or emulsion stability) after the heat treatment in the same manner as described in "Experiment 8; Re-emulsified Composition of Powder Emulsified Composition" above.

To evaluate the aqueous organic solvent resistance of each of the re-emulsified compositions obtained above, ethanol, acetone, or acetonitrile was further added to each of the re-emulsified compositions in an amount of 5 mass%, followed by mixing and stirring. Each of the obtained re-emulsified compositions was evaluated for the demulsified state (or emulsion stability) and the demulsified state (or emulsion stability) after the heat treatment in the same manner as described above.

### (2) Result

**[Table 15]**

| Re-emulsified composition containing 0.01 mass% emulsifier | | | | | | |
|---|---|---|---|---|---|---|
| pH value (25°C) | 2 | 3 | 4 | 7 | 9 | 10 |
| Evaluation of emulsion stability | ⊚ | ⊚ | ⊚ | ⊚ | ⊚ | ⊚ |
| Occurrence of adhesion to the wall surface | Not occurred | Not occurred | Not occurred | Not occurred | Not occurred | Not occurred |
| Evaluation of emulsion stability after heat treatment | ⊚ | ⊚ | ⊚ | ⊚ | ⊚ | ⊚ |

| Re-emulsified composition containing 0.01 mass% emulsifier +5 mass% ethanol | | | | | | |
|---|---|---|---|---|---|---|
| pH value (25°C) | 2 | 3 | 4 | 7 | 9 | 10 |
| Evaluation of emulsion stability | ⊚ | ⊚ | ⊚ | ⊚ | ⊚ | ⊚ |
| Evaluation of emulsion stability after heat treatment | ⊚ | ⊚ | ⊚ | ⊚ | ⊚ | ⊚ |

| Re-emulsified composition containing 0.01 mass% emulsifier +5 mass% acetone | | | | | | |
|---|---|---|---|---|---|---|
| pH value (25°C) | 2 | 3 | 4 | 7 | 9 | 10 |
| Evaluation of emulsion stability | ⊚ | ⊚ | ⊚ | ⊚ | ⊚ | ⊚ |
| Evaluation of emulsion stability after heat treatment | ⊚ | ⊚ | ⊚ | ⊚ | ⊚ | ⊚ |

| Re-emulsified composition containing 0.01 mass% emulsifier +5 mass% acetonitrile | | | | | | |
|---|---|---|---|---|---|---|
| pH value (25°C) | 2 | 3 | 4 | 7 | 9 | 10 |
| Evaluation of emulsion stability | ⊚ | ⊚ | ⊚ | ⊚ | ⊚ | ⊚ |
| Evaluation of emulsion stability after heat treatment | ⊚ | ⊚ | ⊚ | ⊚ | ⊚ | ⊚ |

It was confirmed that the re-emulsified composition having any of the above pH values (25°C), which had been prepared by adding an emulsifier, did not cause demulsification, indicating that even in the presence of an emulsifier, excellent emulsion stability with high pH resistance was maintained.

In addition, no adhesion was observed on the wall surface of the housed glass beaker for the re-emulsified composition having any of the above pH values (25°C), indicating that the adhesion could be suppressed/eliminated by the addition of an emulsifier. Such a re-emulsified composition may be useful, for example, in applications different from those in which adhesion is required, such as, but not limited to, foods and beverages.

It was also confirmed that the re-emulsified composition having any of the above pH values (25°C) maintained an emulsified state after the heat treatment without causing demulsification, indicating that even in the presence of an emulsifier, high heat resistance and excellent emulsion stability were maintained.

Then, it was confirmed that the re-emulsified composition having any of the above pH values (25°C) maintained the emulsified state in the presence of ethanol, acetone, or acetonitrile without causing demulsification and had excellent emulsion stability with high water-soluble organic solvent resistance. In addition, it was confirmed that any of the above re-emulsified compositions to which ethanol, acetone, or acetonitrile was added maintained the emulsified state after heat treatment without causing demulsification, indicating that excellent emulsion stability with high heat resistance was maintained even in the presence of a water-soluble organic solvent.

The results indicates that the re-emulsified composition could be used in combination with a water-soluble organic solvent also used as a solvent or the like, regardless of the pH value or application of a heat treatment.

### Experiment 10: Gustatory Situation Sensory Testing of Re-emulsified Composition

### (1) Preparation of re-emulsified composition of salt-containing powder emulsified composition

According to the composition in Table 16 below, each component was added and mixed to prepare an emulsified composition. Each component was mixed and stirred using a hand blender at 20,000 rpm for 5 minutes. Next, the resulting emulsified composition was freeze-dried (at 20°C for 48 hours) and crushed to obtain a salt-containing powder emulsified composition.

The amount of each component in the table is expressed as an amount of mass%, where the amount of the obtained emulsified composition is 100 mass%.

**[Table 16]**

| | | |
|---|---|---|
| Blending | Fat and oil (palm oil) | 5 |
| | α-cyclodextrin | 4.7 |
| | Water-soluble gelling agent (Xanthan gum) | 0.3 |
| | Sodium chloride | 10 |
| | Water | 80 |
| | Total | 100 |

First, 0.8 g of the resulting salt-containing powder emulsified composition and 99.2 g of water were mixed and stirred to prepare a re-emulsified composition. Saline mixed with 0.4 g of salt and 99.6 g of water was used as a control.

### (2) Sensory Testing Method

Eight routinely trained panelists evaluated the flavor of the resulting re-emulsified composition according to the following procedures 1 to 8.
1. The panelists rinsed the mouth with an appropriate amount of water and spat it out.
2. The panelists took 10 g of the control saline and spread it throughout the mouth.
3. The sensory evaluation at that time was used as the first impression to evaluate the intensity of saltiness by the VAS method.
4. After the first impression evaluation, the panelists swallowed the control saline, and one minute later, evaluated the intensity of saltiness as a second impression by the VAS method.
5. The panelists rinsed the mouth with an appropriate amount of water.
6. The panelists took 10 g of the re-emulsified composition of the salt-containing powder emulsified composition and spread it throughout the mouth.
7. The sensory evaluation at that time was used as the first impression to evaluate the intensity of saltiness by the VAS method.
8. After the first impression evaluation, the panelists swallowed the re-emulsified composition, and one minute later, evaluated the intensity of saltiness as a second impression by the VAS method.

### (3) Result

The evaluation results of the first impression and second impression of the control saline and the re-emulsified composition by the respective panelists are shown in Table 17 below. Furthermore, the evaluation results are shown in the following Table 18 in which the values of the first impression of the control saline were set to "100%" and the respective values were expressed as relative values, and in the following Table 19 in which the values of the second impression of the control saline were set to "100%" and the values of the second impression of the re-emulsified composition were expressed as relative values.

**[Table 17]**

| Panelist No. | Control saline | | Re-emulsified composition of salt-containing powder emulsified composition | | Note |
|---|---|---|---|---|---|
| | First impression | Second impression | First impression | Second impression | |
| 1 | 6.6 | 0.5 | 5.6 | 2.4 | The re-emulsified composition was mellow. The re-emulsified composition spread throughout the mouth. |
| 2 | 6.7 | 2 | 4.3 | 1.6 | |
| 3 | 6.5 | 1.8 | 5 | 0.8 | The re-emulsified composition was mild. |
| 4 | 7.3 | 1.9 | 8.3 | 2.5 | The re-emulsified composition had a sea salt-like off-flavor and mineral feeling. |
| 5 | 2.1 | 0.3 | 4 | 1.5 | |
| 6 | 6.5 | 1.9 | 5 | 1.5 | The re-emulsified composition expanded in the mouth. |
| 7 | 8 | 0.1 | 5.7 | 3.3 | |
| 8 | 8 | 0.1 | 9.6 | 0.3 | |

**[Table 18]**

| Panelist No. | Control saline | | Re-emulsified composition of salt-containing powder emulsified composition | | Note |
|---|---|---|---|---|---|
| | First impression | Second impression | First impression | Second impression | |
| 1 | 100% | 7.6% | 84.8% | 36.4% | The re-emulsified composition was mellow. The re-emulsified composition spread throughout the mouth. |
| 2 | 100% | 29.9% | 64.2% | 23.9% | |
| 3 | 100% | 27.7% | 76.9% | 12.3% | The re-emulsified composition was mild. |
| 4 | 100% | 26.0% | 113.7% | 34.2% | The re-emulsified composition had a sea salt-like off-flavor and mineral feeling. |
| 5 | 100% | 14.3% | 190.5% | 71.4% | |
| 6 | 100% | 29.2% | 76.9% | 23.1% | The re-emulsified composition expanded in the mouth. |
| 7 | 100% | 1.3% | 71.3% | 41.3% | |
| 8 | 100% | 1.3% | 120.0% | 3.8% | |

**[Table 19]**

| Panelist No. | Control saline | | Re-emulsified composition of salt-containing powder emulsified composition | | Note |
|---|---|---|---|---|---|
| | First impression | Second impression | First impression | Second impression | |
| 1 | - | 100% | - | 480.0% | The re-emulsified composition was mellow. The re-emulsified composition spread throughout the mouth. |
| 2 | - | 100% | - | 80.0% | |
| 3 | - | 100% | - | 44.4% | The re-emulsified composition was mild. |
| 4 | - | 100% | - | 131.6% | The re-emulsified composition had a sea salt-like off-flavor and mineral feeling. |
| 5 | - | 100% | - | 500.0% | |
| 6 | - | 100% | - | 78.9% | The re-emulsified composition expanded in the mouth. |
| 7 | - | 100% | - | 3300.0% | |
| 8 | - | 100% | - | 300.0% | |

It was confirmed that the re-emulsified composition of the salt-containing powder emulsified composition tended to have a stronger salty taste in each of the first impression and the second impression, as compared to the control saline. In particular, the second impression showed a stronger salty taste as compared to the control saline. The results confirmed that by using the emulsified composition as a carrier for an active component, the active component can be slowly released from the composition, and the active component could be made to act or sensed for a longer period of time.

### Experiment 11: Performance Evaluation of Emulsified Composition (Imparting Hydrophilic Surface)

### (1) Test method

In "Experiment 6: Evaluation of Heat Resistance of Powder Emulsified Composition" above, the emulsified composition of Example 23 prepared according to the composition in Table 8 was used in the following test.

After any of the following treatments 1. to 3. was performed on the surface of polyethylene resin (5 cm × 5 cm × 1 mm (thickness)), 100 µL of distilled water was dropped to compare the angle formed by the droplet surface and the resin surface (contact angle).
1. 1 mL of 100% ethanol was dropped into the center of the polyethylene resin, which was evenly coated by applying an appropriate pressure with a vinyl gloved hand. The excess ethanol was lightly wiped off with Kimwipes and then dried.
2. 1 mL of the emulsified composition of Example 23 was dropped into the center of the polyethylene resin, which was evenly coated by applying an appropriate pressure with a vinyl gloved hand. The excess emulsified composition was lightly wiped off with Kimwipes and then dried.
3. The emulsified composition of Example 23 was applied to the center of the polyethylene resin in the same manner as in 2. above and dried, and then 100% ethanol was applied thereto and dried in the same manner as in 1. above.

### (2) Result

Figure 1 shows the state of the liquid droplets dropped on the surface of the polyethylene resin that has been subjected to each treatment. The magnitude of the contact angle in distilled water droplets dropped on the surface of the polyethylene resin that had been subjected to Treatment 3. was larger than that in the other resins, confirming that the surface of the polyethylene resin had good wettability (i.e., high hydrophilicity). It is suggested that the hydrophilic part of the emulsified composition remained on the surface of the polyethylene resin by wiping with 100% ethanol after the emulsified composition of Example 23 was applied through Treatment 3., thereby enhancing the hydrophilicity of the surface.

### Experiment 12: Performance Evaluation of Emulsified Composition (Smoothing of Skin Surface)

### (1) Test method

In "Experiment 6: Evaluation of Heat Resistance of Powder Emulsified Composition" above, the emulsified composition of Example 23 prepared according to the composition in Table 8 was used in the following test.

The whole hand of the subject was thoroughly washed with a hand soap to remove oil on the skin surface as much as possible. Although the same result can be obtained when only water is used or when soaps such as a hand soap is used for washing, the hand soap was used for washing in this experiment.

The surface of the pad of the index finger of the left hand was subjected to any of the following treatments 1. to 3., and then the skin of the surface was observed with a stereomicroscope, followed by a sensory examination of the feeling.
1. Untreated: wind was continuously applied to the finger pad side of the left index finger with a blowing function of a dryer for 10 minutes.
2. The emulsified composition of Example 23 was applied to the surface of the finger pad side of the left index finger, then lightly wiped, and was continuously blown with the blowing function of the dryer for 10 minutes.
3. The emulsified composition of Example 23 was applied to the surface of the finger pad side of the left index finger, thoroughly washed with the hand soap, lightly wiped, and then continuously blown with the blowing function of the dryer for 10 minutes.

### (2) Result

As a result of stereomicroscopic observation of the finger pad side of the index finger of the left hand subjected to each treatment, it was confirmed that in the case where the emulsified composition of Example 23 was applied to the skin surface (treatment 2., treatment 3.), the unevenness and wrinkles of the surface were alleviated and the surface became smoother than in the case of no treatment (treatment 1.). This effect was more significantly confirmed when the emulsified composition of Example 23 was applied and then washed (treatment 3.).

The sensory test results also showed similar results: the skin surface was hard and felt more resistant and rough when untreated (treatment 1.), but when the emulsified composition of Example 23 was applied to the skin surface (treatment 2., treatment 3.), the skin surface remained moist and felt silky and smooth.

The results suggested that the emulsified composition obtained by blending cyclodextrin and a prescribed water-soluble gelling agent in combination in an oil-in-water emulsion containing water and fat and oil, the dried form obtained by subjecting the same to a drying treatment (powder emulsified composition), and the re-emulsified composition in the dried form have excellent stability with various resistances and can be used in various applications.

## Claims

1. An emulsified composition, comprising water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum, wherein a ratio of a blending amount of the fat and oil to a total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio.

2. The emulsified composition according to claim 1, wherein a content of the water is 10 mass% or less.

3. The emulsified composition according to claim 1 or 2, which has a powder form.

4. A re-emulsified composition, comprising the emulsified composition according to claim 2 or 3 and a solvent.

5. The re-emulsified composition according to claim 4, further comprising an emulsifier.

6. The re-emulsified composition according to claim 4 or 5, further comprising an aqueous organic solvent.

7. A method for producing an emulsified composition, comprising mixing water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum to form an emulsified composition such that a ratio of a blending amount of the fat and oil to a total blending amount of the cyclodextrin and the water-soluble gelling agent (blending amount of the fat and oil: total blending amount of the cyclodextrin and the water-soluble gelling agent) is in a range of 1 or more and less than 9:9 or less and more than 1 in mass ratio.

8. The method for producing an emulsified composition according to claim 7, further comprising subjecting the obtained emulsified composition to a drying treatment.

9. The method for producing an emulsified composition according to claim 8, wherein a content of the water in the emulsified composition is adjusted to 10 mass% or less by the drying treatment.

10. The method for producing an emulsified composition according to claim 8 or 9, further comprising providing the obtained emulsified composition in a powder form.

11. A method for producing a re-emulsified composition, comprising mixing the emulsified composition according to claim 2 or 3 with a solvent to form a re-emulsified composition.

12. The method for producing a re-emulsified composition according to claim 11, further mixing an emulsifier.

13. The method for producing a re-emulsified composition according to claim 11 or 12, further mixing an aqueous organic solvent.
